# EUROPEAN PATENT SPECIFICATION

(11) **EP 2 015 715 B1**
(45) Date of publication and mention of the grant of the patent: **02.01.2013**
(21) Application number: 07732729.4
(22) Date of filing: 08.05.2007
(51) Int. Cl.: A61F 5/01

(54) **SUPPORT DEVICE**
STÜTZVORRICHTUNG
DISPOSITIF DE SUPPORT

(30) Priority: 11.05.2006 GB 0609380
(43) Date of publication of application: 21.01.2009
(73) Proprietor: MacFarlane, Doniert, London SE24 0DX (GB)
(72) Inventor: MacFarlane, Doniert, London SE24 0DX (GB)
(74) Representative: Bennett, Adrian Robert J.
(86) International application number: PCT/GB2007/001701
(87) International publication number: WO 2007/132181

(56) References cited:
- US-A- 5 772 620
- US-A1- 2005 101 898
- US-A1- 2005 267 391
- US-B1- 6 200 286

## Description

The invention relates to an orthopaedic support device for supporting, protecting and immobilising part of a hand, and particularly, but not exclusively, to a 5^{th} Metacarpal fracture splint.

The hand is made up of many bones, all of which are susceptible to fracture. There are five Metacarpal bones which make up the palm of the hand, each one connecting a finger or thumb to the bones in the wrist. The Metacarpal bones are numbered 1 to 5, the 1^{st} Metacarpal being associated with the thumb, and the 5^{th} Metacarpal being associated with the 4^{th}, or little finger. The 5^{th} Metacarpal fracture is one of the most commonly seen hand fractures. It occurs at the neck of the bone close to the knuckle, often as a result of striking something or someone, and is therefore commonly referred to as 'boxer fracture'.

There is currently no clear consensus within the medical profession as to the best way of setting these fractures; although it is widely agreed that some degree of immobilisation of the broken bone is beneficial.

In extreme cases, where there is a misalignment of the portions of the bone, surgery may be required to ensure that the fracture sets correctly. More commonly, the hand will simply be splinted to support the broken bone during the healing process.

Typically, a cast made of plaster of paris or fibreglass is formed around the hand, wrist and forearm of the patient. The hand is set in a fixed position, often with the fingers and palm in 90 degree flexion because this has been thought to be the preferred angle for healing. This is no longer universally accepted however. As an alternative to the cast, a forearm based splint may be applied under a compressive wrap. Casts have the advantage that the hand is well supported and protected during treatment, however the cast itself often proves inconvenient and uncomfortable for the wearer and may also, over the typical treatment duration of 4 to 6 weeks, cause allergies and joint stiffness. Swelling of the hand within the rigid cast can also lead to pressure and further discomfort for the wearer. In extreme cases, this can require visits to a hospital for evaluation. Furthermore, many medical professionals now believe that allowing movement of the hand during the course of treatment is beneficial, not only in reducing stiffness in the joints, but also in encouraging better bone healing, this movement is not possible with typical casts.

Another drawback of casting is that the process of forming the cast is time consuming and often calls for a specialist, which can result in long waiting times in A&E departments. While a broken bone is not properly immobilised, there exists the possibility of further injury and damage to the surrounding muscles, blood vessels, tendons and nerves. It is therefore beneficial if the fracture is immobilised as quickly as possible.

A more simple approach is to merely tape the 4^{th}, or little finger, to the 3^{rd}, or ring finger thereby using one finger to splint the other. This provides a degree of support to the fractured Metacarpal while allowing continued functionality of the injured hand. Despite the simplicity of this approach, it has been shown to be effective. However, the fractured bone is provided no protection against accidental knocks which can be extremely painful. Furthermore, a common problem with Metacarpal fractures is that, during setting, the head of the bone (the knuckle) can become depressed resulting in a bump on the palm of the hand. This can cause discomfort when the hand is used, especially when gripping articles tightly.

US 6,953,441 describes a preformed brace for treating 4^{th} and 5^{th} Metacarpal fractures which is attachable to the hand and wrist of the wearer by a number of straps. The brace consists of a semi rigid one-piece outer shell, which extends from above the wrist to the ends of the fingers along the outside of the hand and wrist of the wearer. The brace therefore only provides support to the outside of the wrist and hand, limiting the amount of support that can be provided. The brace places the wrist of the wearer in extension, with the fingers immobilised in flexion at an angle of 80 to 90 degrees. With the fingers immobilised at such a large angle there is substantial loss of functionality of the hand.

It is an object of the present invention to combine the benefits of the aforementioned methods while removing the associated drawbacks. In other words it is an object of the present invention to provide a comfortable lightweight and easily fitted support device that nonetheless provides improved support and protection for fractures.

US 5,772,620 relates to a support device for the treatment of tendovaginitis. The device comprises a fabric sleeve and a splint which is inserted into a pocket in the sleeve to provide support to a user's hand and wrist.

According to the present invention there is provided an orthopaedic support device according to the appended claim 1. Further advantageous features are recited in the associated dependent claims.

In general terms the invention has many advantages over the previously described support devices. It is shaped so as to provide not only support, but also impact protection to the broken bone while allowing the patient continued use of the hand for day to day activities such as writing. Beneficial flexing and extension of the hand and wrist can be achieved by the support, and by limiting the angles to considerably less than 90 degrees a far greater degree of functionality of the hand can be maintained. If the thumb, index finger and middle finger are left free by the device, the 'tripod' grip used in fine movements of the hand is possible. Preferably a substantial part of the width of the wrist and hand is supported by the device. In particular support should be provided for the 1^{st} to 3^{rd} Metacarpal as this is believed to be beneficial in reducing the deforming forces on the fracture site. This additional support also allows advantageous positioning of the wrist and hand in more than one plane. For example the support may be designed to place the hand at an angle in the lateral direction as well as the longitudinal direction.

Being preformed the support device can be fitted to a patient quickly and easily by a relatively unskilled person. This will minimise waiting times at A&E departments and should also enable use of the device at the time and place of the injury, for example on she sports field. This is clearly beneficial since immobilising the fracture minimises the chances of any further damage to the hand and also reduces the pain and discomfort of the patient.

Means for cooling the skin may be provided. These are preferably ventilating means, preferably comprising apertures or perforations in the support member.

In a preferred embodiment, the support member is provided with releasable straps for fitting. The support is therefore easily removable for hand physiotherapy or cleaning (one of the main gripes of plaster of paris immobilisation). The patient is also able to remove and reapply the splint themselves during the course of treatment, either to allow the aforementioned beneficial movement of the hand, or simply to allow completely unrestricted use of the hand for short periods when greater dexterity is required.

The support device will advantageously be provided in a number of different sizes, and/or be provided with adjustable straps. This allows the device to be preformed but still be applicable to different sized patients. It is anticipated that the devices will range in size from 11 cm to 41 cm in length and from 5cm to 10cm in width. The provision of adjustable straps also permits some fine tuning of the fit of the device as may be required due to increased or decreased swelling of the injured hand. Such adjustment is not possible with a moulded cast, which would cause pressure, and lose its fit to the hand respectively.

Embodiments of the present invention will now be described in the following detailed description, given by way of example only, with reference to the accompanying drawings, in which:
Figure 1 is a top view of a support device according to a first embodiment;
Figures 2, 3 and 4 are lateral cross-sectional views of the support device of Figure 1 taken at the lines marked in Figure 1 as II, III and IV respectively;
Figures 5 and 6 are longitudinal cross-sectional views of the support device of Figure 1;
Figure 7 is a perspective view of the support device of Figure 1, taken from the wrist end towards the fingers;
Figure 8 is a top view of a preferred arrangement of straps for attaching the support device of Figure 1 to a patient;
Figure 9 is a top view of a support device not according to the present invention;
Figures 10, 11 and 12 are lateral cross-sectional views of the support device of Figure 9 taken at the lines marked in Figure 9 as X, XI and XII respectively;
Figure 13 is a side view of the support device of Figure 9 taken from the thumb side towards the outside of the hand;
Figure 14 is a perspective view of the support device of Figure 9, viewed from the wrist end towards the fingers; and
Figures 15 and 16 are side and top views respectively of a preferred arrangement of straps for attaching the support device of Figure 9 to a patient.

Although all Figures show a device suitable for supporting the right hand and wrist of a patient, it will be understood that similar devices for the left hand are simply mirror images of those shown.

For convenience the dimensions and angles indicated by reference letters in the accompanying drawings are set out in the following tables.

### Figures 1-8:

| **Dimensions (cm)** | | | **Angles (°)** | | |
|---|---|---|---|---|---|
| **ID** | **Minimum** | **Maximum** | **ID** | **Angle** | **Variance** |
| A | 3 | 7 | a | 25 | 20 |
| B | 5 | 10 | b | 50 | 20 |
| C | 0.5 | 4 | c | 60 | 20 |
| D | 4 | 11 | d | 45 | 20 |
| E | 0.5 | 2 | e | 90 | 20 |
| F | 2 | 5 | f | 15 | 20 |
| G | 2 | 6 | g | 45 | 20 |
| H | 3 | 9 | ( from the horizontal) | | |
| I | 1 | 9 | h | 25 | 20 |
| J | 1 | 3 | (from the horizontal) | | |
| K | 0.5 | 3 | i | 40 | 20 |
| L | 5 | 12 | j | 35 | 20 |
| M | 4 | 8 | i | 40 | 20 |
| N | 2 | 7 | j | 35 | 20 |
| Q | 1 | 4 | | | |
| R | 5 | 9 | | | |

### Figures 9-16:

| **Dimensions (cm)** | | | **Angles (°)** | | |
|---|---|---|---|---|---|
| **ID** | **Minimum** | **Maximum** | **ID** | **Angle** | **Variance** |
| A' | 6 | 12 | a' | 25 | 20 |
| B' | 6 | 13 | b' | 20 | 20 |
| C' | 2 | 9 | c' | 30 | 20 |
| D' | 6 | 16 | d' | 35 | 20 |
| E' | 12 | 25 | e' | 70 | 20 |
| F' | 5 | 7 | f | 10 | 20 |
| G' | 7 | 11 | g' | 18 | 20 |
| H' | 3 | 5 | | | |
| I' | 5 | 10 | | | |
| J' | 3 | 6 | | | |
| K' | 2 | 6.5 | | | |
| L' | 3 | 8 | | | |
| M' | 4 | 10 | | | |
| N' | 6 | 15 | | | |
| P' | 4 | 11 | | | |
| Q' | 6 | 10 | | | |
| R' | 1 | 3 | | | |
| S' | 4 | 8 | | | |
| T | 6 | 9 | | | |
| U' | 6 | 11 | | | |

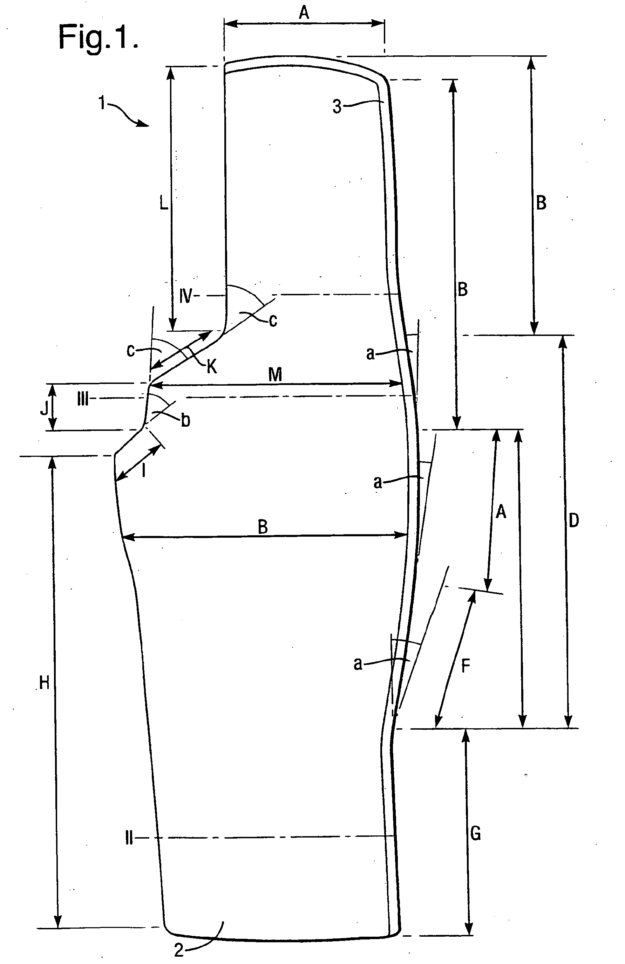

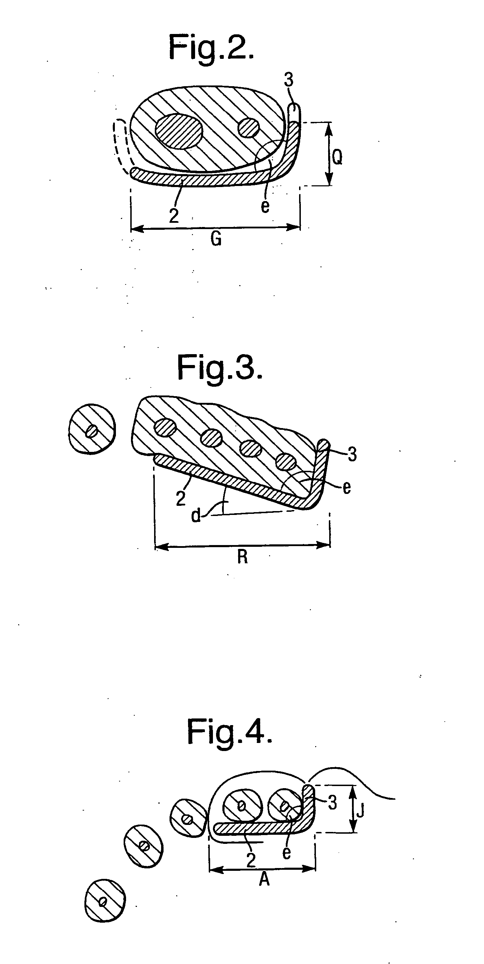

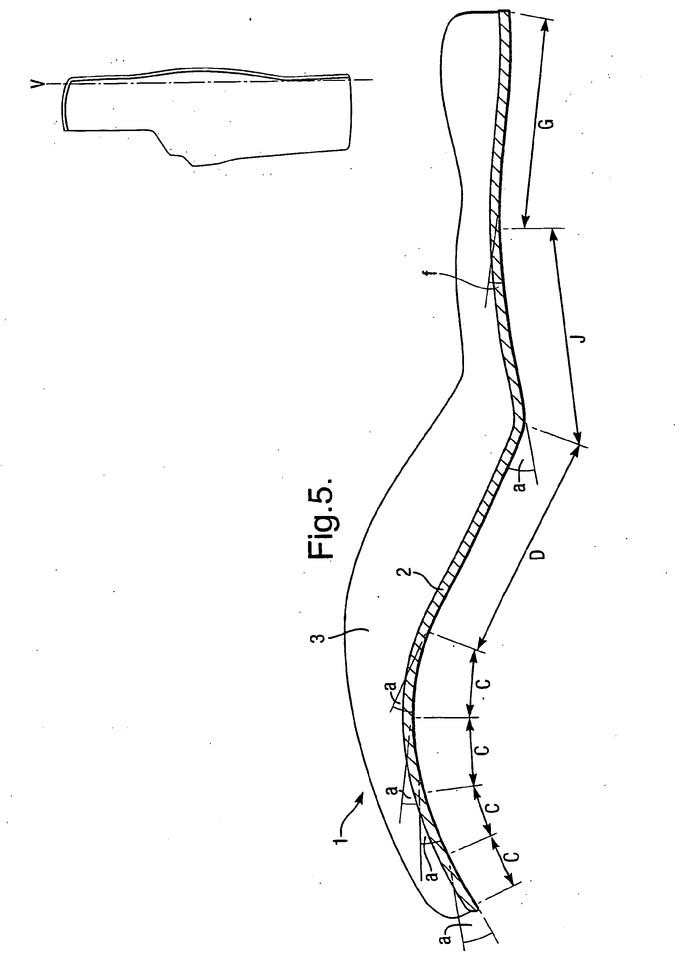

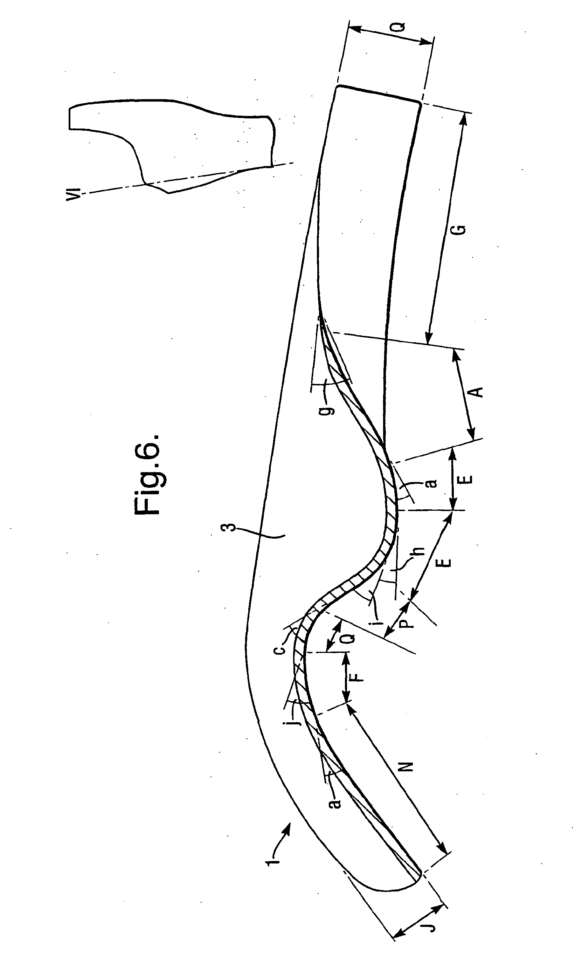

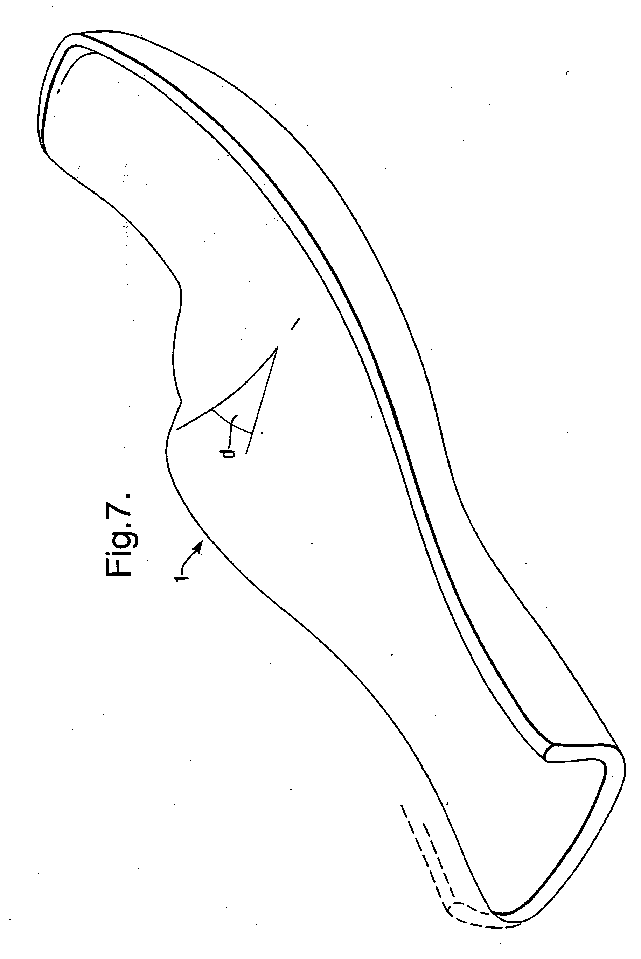

An embodiment of the invention, as shown in Figures 1-8, is particularly appropriate for setting undisplaced fractures of the 5^{th} Metacarpal bone. The support member (1) shown in Figure 1 is between 11cm and 27 cm in overall length, and has a maximum width of between 5cm and 10cm to accommodate different sized patients. In use, it extends along the underside of the patient's arm from just above the wrist, down the hand and to the end of the 3^{rd} and 4^{th} fingers. As shown in Figures 2, 3 and 4, the support member (1) has an approximately 'L' shaped cross-section, the longer more horizontal part (2) providing the support under the patient's arm and hand with the shorter more vertical part (3) on the outside of the hand to provide protection for the fractured 5^{th} Metacarpal bone. In this particular embodiment the support member (1) is shaped in a longitudinal direction so as to place the wrist in extension at an angle (a) between 25° and 65°, preferably at 45° and the fingers in flexion at a similar but opposite angle, as best shown in Figure 5. The support also features a slope in the lateral direction, which places the hand at an angle so that the thumb is in an elevated position relative to the outside of the hand. This angle (d) is best shown in Figures 3 and 7, and is between 25° and 65 °, preferably around 45 °. A common problem with this type of injury is a lower position of the 4^{th} knuckle after the Metacarpal fracture sets. Positioning the hand as described helps to maintain a high position of the 4^{th} knuckle during the bone setting.

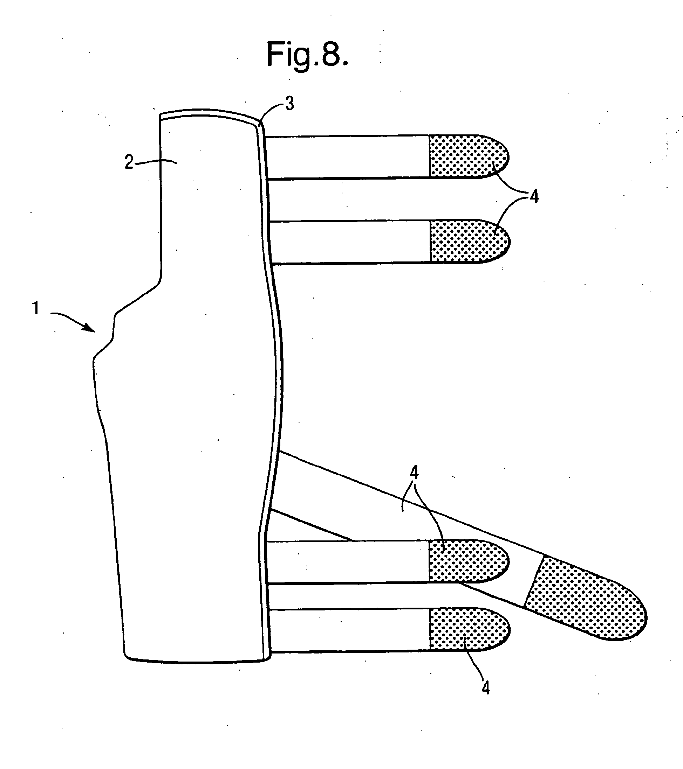

It is clear from the drawings that only the bottom portion of the hand and wrist is covered by the device. Swelling on the top of the hand is unimpeded by the support which is beneficial in preventing skin necrosis. The hand and wrist can also breaths more freely, this reduces the possibility of unwelcome allergic reactions. To further aid breathing ventilation means, for example apertures or perforations (not shown) may be provided in the support member. The device is also lightweight and is provided with straps (4) as shown in Figure 8, so as to be easily removable and refittable if required. The use of adjustable and releasable straps (4) allows the wearer to loosen the device slightly if swelling of the hand is causing discomfort. As the swelling goes down in the course of treatment (typically days 5-7) the straps (4) can be tightened to take any slack and allow the device to provide the best possible support. This makes it far less of an inconvenience for the wearer than a traditional cast or splint.

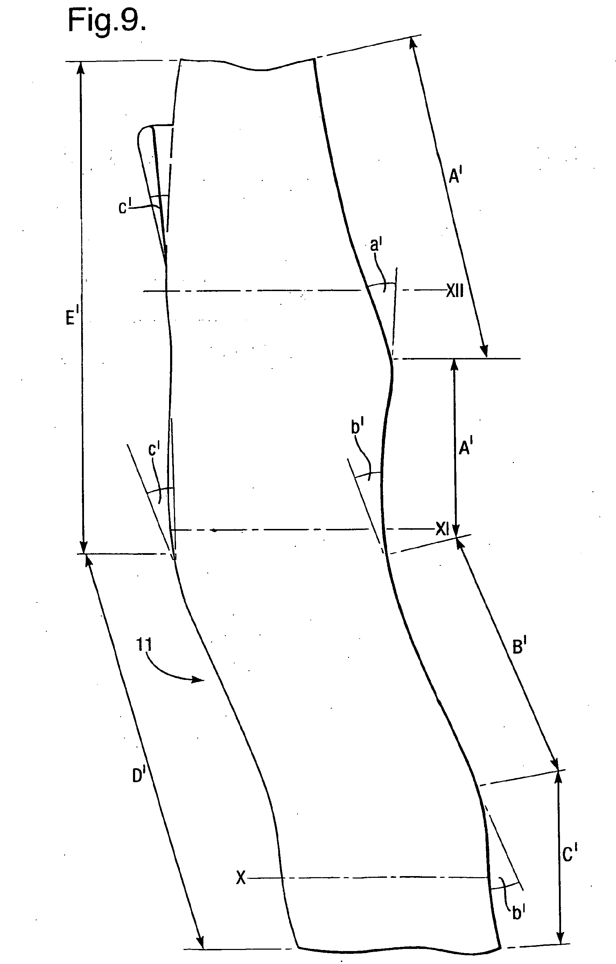

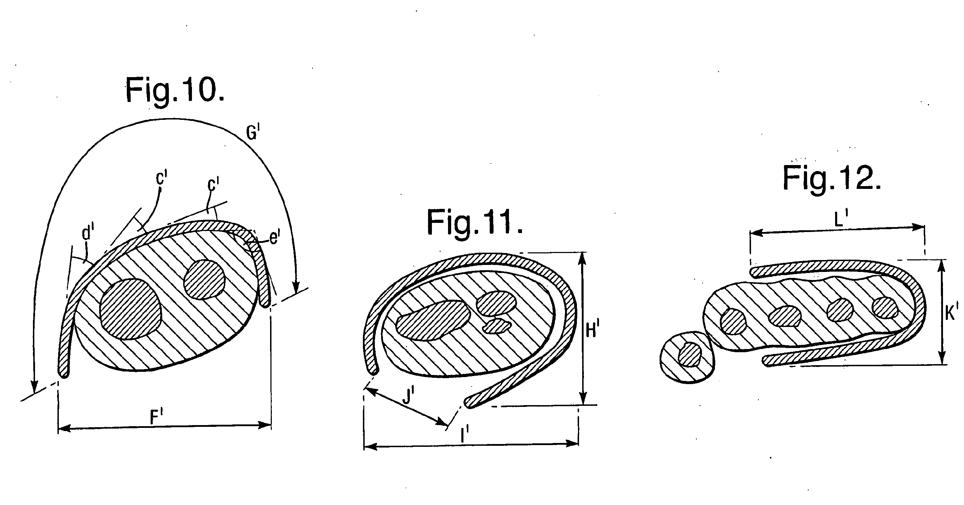

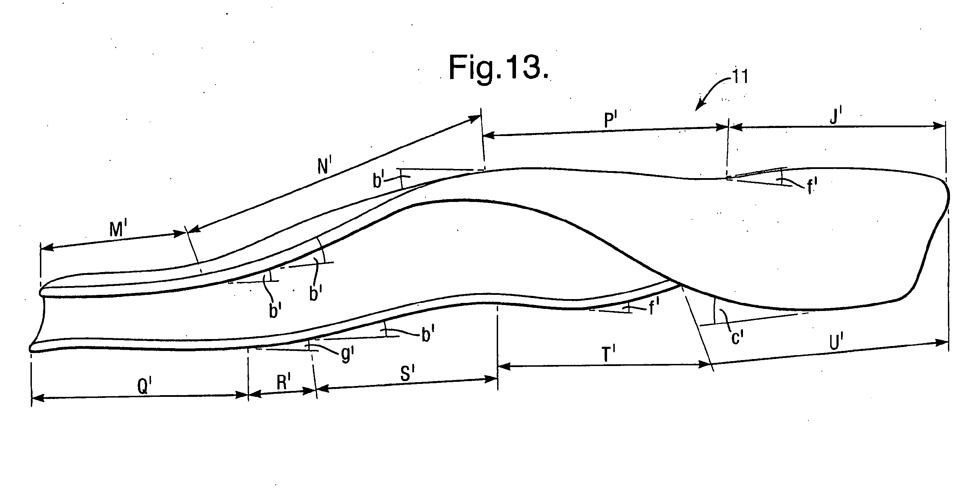

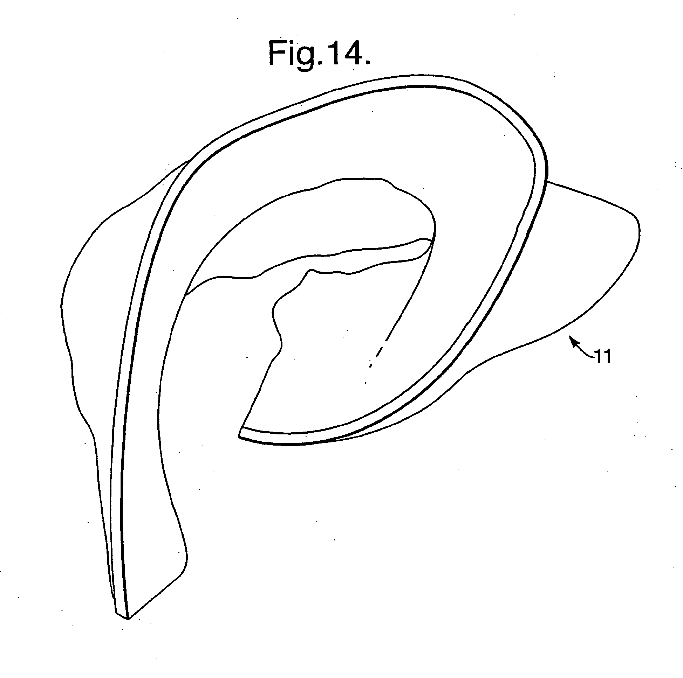

The device of Figures 9 to 16 is not in accordance with the present invention, but is particularly suited to setting displaced fractures of the 5^{th} Metacarpal bone. The support member (11) shown in Figure 9 is between 5cm and 10cm wide, and between 18cm and 41 cm in length. As a result, it extends further along the patient's arm than the support member (1) of the first embodiment, the position of the wrist being approximately at the section line XI. Figure 9 also shows that the support provides angulation to the wrist and hand as viewed from above. The support member (11) takes the form of a spiral or a twisted 'C' shaped cross-section. Figure 13 shows this shape viewed from the side, while Figure 14 is a view from the wrist end looking towards the fingers. When applied, the support covers the top and sides of the forearm and most of the wrist, then twists through approximately 90 degrees to cover and protect the 4^{th} and 5^{th} Metacarpal bones on the outside of the hand and to support the 3^{rd} and 4^{th} fingers. This twisting is shown in the cross-sectional views of Figures 10, 11 and 12. Figure 13 shows that the support member (11) according to this particular example is shaped in a longitudinal direction so as to place the wrist in flexion and the fingers in extension. Flexing the wrist reduces deforming forces on the flexor tendons, while extending the fingers serves to reduce the angle of deformity. The soft tissue of the hand in this arrangement will help to reduce the fracture leading to a better set of the bone.

The twisted 'C' shape cross-section allows the support member (11) to surround the hand and wrist more completely than would otherwise be the case. As a result, the support of this example advantageously offers a greater number of possible support positions for the hand and wrist. Although Figures 9 to 16 show the support in a configuration to place the wrist in flexion and the fingers in extension, there is no reason why it could not be configured to place the wrist in extension with the fingers in flexion as in the previous embodiment.

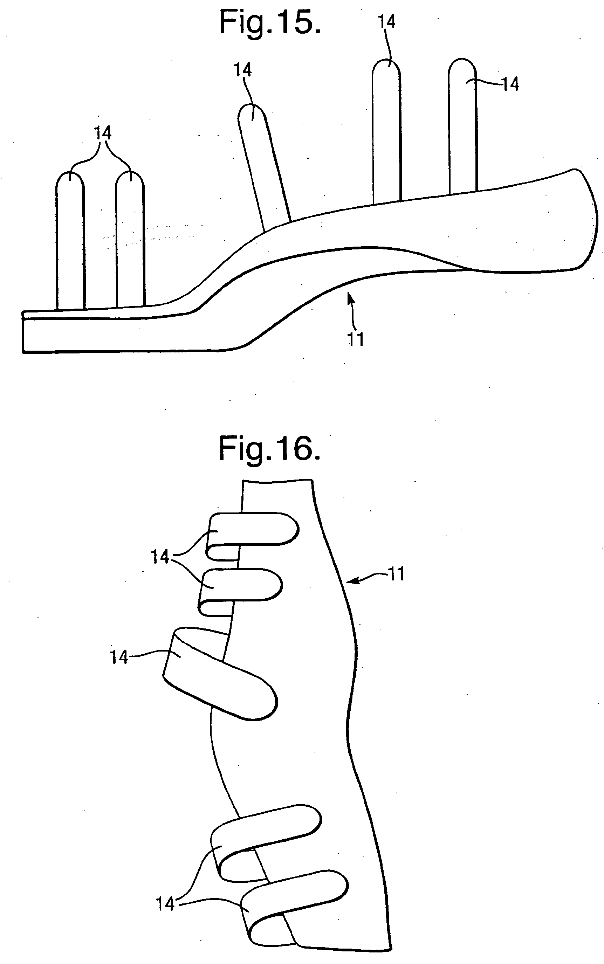

Although more of the hand is covered than in the previous embodiment, a significant portion still remains uncovered, and is able to breathe freely. Ventilation means (not shown) may be provided as before. As before, the device is lightweight and is easily adjustable, removable and refittable, due to the provision of releasable and adjustable straps (14) as shown in Figure 16.

While the above descriptions relate to fractures of the 5^{th} Metacarpal, it will be apparent to one skilled in the art that alternative embodiments exist within the scope of the claims that would also make the invention suitable for the treatment of other hand and wrist fractures, for example variations of the device could be made to support other fingers in a similar fashion.

The present invention is not limited to the specific embodiment described above. Alternative arrangements will be apparent to a reader skilled in the art.

## Claims

1. An orthopaedic support device for supporting, protecting and immobilising part of a hand, the device comprising a preformed support member (1) which, in use, extends along the wearer's wrist and hand, and is attachable thereto, so as to provide support with the hand disposed in one direction so that the wrist joint is at a given angle, and the fingers disposed in another direction so that the knuckles are at an angle substantially similar in magnitude but opposite in orientation to the wrist joint and not exceeding 55 degrees, **characterised in that** the preformed support member (1) is substantially 'L' shaped in cross-section.

2. An orthopaedic support device according to claim 1, wherein the preformed support member (1) is attachable to the wearer by at least one strap (4).

3. An orthopaedic support device according to claim 2, wherein the at least one strap (4) is releasable.

4. An orthopaedic support device according to claim 2 or 3, wherein the at least one strap (4) is adjustable.

5. An orthopaedic support device according to any one of the preceding claims, wherein the preformed support member (1) is shaped to provide protection for the outside of the hand.

6. An orthopaedic support device according to any one of the preceding claims, wherein the preformed support member (1) is shaped to provide support across a substantial part of the width of the wrist and hand.

7. An orthopaedic support device according to claim 6, wherein the preformed support member (1) is shaped to support the hand and wrist at predetermined angles in more that one plane.

8. An orthopaedic support device according to any one of the preceding claims wherein the index finger, middle finger and thumb are left free.

9. An orthopaedic support device according to any previous claim, wherein the preformed support member (1) is shaped so as to place the wearer's wrist in extension and the wearer's fingers in flexion.

10. An orthopaedic support device according to any of the preceding claims, wherein the preformed support member (1) is shaped to support the hand at an angle in the lateral direction.

11. An orthopaedic support device according to claim 10, wherein the lateral angle is between 25 and 65 degrees.

12. An orthopaedic support device according to claim 10 or 11, wherein the hand is supported so that the thumb is elevated in relation to the outside of the hand.

13. An orthopaedic support device according to any one of the preceding claims, wherein ventilating means are provided.

## Patentansprüche

1. Orthopädische Stützvorrichtung zum Stützen, Schützen und Ruhigstellen eines Teils einer Hand, wobei die Vorrichtung ein vorgeformtes Stützelement (1) umfasst, das sich im Gebrauch entlang des Handgelenks und der Hand des Trägers erstreckt und daran angebracht werden kann, um eine Stützung bereitzustellen, wobei die Hand derart in einer Richtung angeordnet ist, dass das Handgelenk sich in einem gegebenen Winkel befindet, und die Finger derart in einer anderen Richtung angeordnet sind, dass die Knöchel sich in einem Winkel befinden, der in Bezug auf die Größenordnung dem Handgelenk im Wesentlichen ähnlich ist, jedoch eine entgegengesetzte Ausrichtung zu diesem aufweist und nicht 55 Grad übersteigt, **dadurch gekennzeichnet, dass** das vorgeformte Stützelement (1) einen im Wesentlichen "L"-förmigen Querschnitt aufweist.

2. Orthopädische Stützvorrichtung nach Anspruch 1, wobei das vorgeformte Stützelement (1) durch mindestens einen Gurt (4) an dem Träger angebracht werden kann.

3. Orthopädische Stützvorrichtung nach Anspruch 2, wobei der mindestens eine Gurt (4) lösbar ist.

4. Orthopädische Stützvorrichtung nach Anspruch 2 oder 3, wobei der mindestens eine Gurt (4) verstellbar ist.

5. Orthopädische Stützvorrichtung nach einem der vorhergehenden Ansprüche, wobei das vorgeformte Stützelement (1) derart geformt ist, dass es einen Schutz für die Außenseite der Hand bereitstellt.

6. Orthopädische Stützvorrichtung nach einem der vorhergehenden Ansprüche, wobei das vorgeformte Stützelement (1) derart geformt ist, dass es eine Stützung über einen wesentlichen Teil der Breite des Handgelenks und der Hand bereitstellt.

7. Orthopädische Stützvorrichtung nach Anspruch 6, wobei das vorgeformte Stützelement (1) derart geformt ist, dass es die Hand und das Handgelenk in vorherbestimmten Winkeln in mehr als einer Ebene stützt.

8. Orthopädische Stützvorrichtung nach einem der vorhergehenden Ansprüche, wobei der Zeigefinger, der Mittelfinger und der Daumen frei gelassen werden.

9. Orthopädische Stützvorrichtung nach einem vorhergehenden Anspruch, wobei das vorgeformte Stützelement (1) geformt ist, um das Handgelenk des Trägers in Extension und die Finger des Trägers in Flexion zu bringen.

10. Orthopädische Stützvorrichtung nach einem der vorhergehenden Ansprüche, wobei das vorgeformte Stützelement (1) derart geformt ist, dass die Hand in einem Winkel in der seitlichen Richtung gestützt wird.

11. Orthopädische Stützvorrichtung nach Anspruch 10, wobei der seitliche Winkel zwischen 25 und 65 Grad liegt.

12. Orthopädische Stützvorrichtung nach Anspruch 10 oder 11, wobei die Hand derart gestützt wird, dass der Daumen im Verhältnis zu der Außenseite der Hand angehoben ist.

13. Orthopädische Stützvorrichtung nach einem der vorhergehenden Ansprüche, wobei Belüftungsmittel vorgesehen werden.

## Revendications

1. Dispositif de support orthopédique pour supporter, protéger et immobiliser une partie d'une main, ce dispositif comprenant un élément de support préformé (1), qui, en cours d'utilisation, s'étend le long du poignet et de la main du porteur, et qui peut être attaché à ceux-ci, de façon à fournir un support avec la main disposée dans une direction de manière à ce que l'articulation du poignet soit à un angle donné, et avec les doigts disposés dans une autre direction de façon à ce que les jointures des doigts soient à un angle d'une grandeur essentiellement similaire mais d'une orientation opposée à l'articulation du poignet et ne dépassant pas 55 degrés, **caractérisé en ce que** l'élément de support préformé (1) a essentiellement une coupe transversale en forme de 'L'.

2. Dispositif de support orthopédique selon la revendication 1, dans lequel l'élément de support préformé (1) peut être attaché au porteur par au moins une courroie (4).

3. Dispositif de support orthopédique selon la revendication 2, dans lequel l'au moins une courroie (4) peut être libérée.

4. Dispositif de support orthopédique selon la revendication 2 ou 3, dans lequel l'au moins une courroie (4) est réglable.

5. Dispositif de support orthopédique selon l'une quelconque des revendications précédentes, dans lequel l'élément de support préformé (1) est formé de façon à fournir une protection pour l'extérieur de la main.

6. Dispositif de support orthopédique selon l'une quelconque des revendications précédentes, dans lequel l'élément de support préformé (1) est formé de façon à fournir un support de part en part d'une partie importante de la largeur du poignet et de la main.

7. Dispositif de support orthopédique selon la revendication 6, dans lequel l'élément de support préformé (1) est formé de façon à supporter la main et le poignet à des angles prédéterminés dans plus d'un plan.

8. Dispositif de support orthopédique selon l'une quelconque des revendications précédentes, dans lequel l'index, le majeur et le pouce sont laissés libres.

9. Dispositif de support orthopédique selon l'une quelconque des revendications précédentes, dans lequel l'élément de support préformé (1) est formé de façon à placer le poignet du porteur en extension et les doigts du porteur en flexion.

10. Dispositif de support orthopédique selon l'une quelconque des revendications précédentes, dans lequel l'élément de support préformé (1) est formé de façon à supporter la main à un angle dans la direction latérale.

11. Dispositif de support orthopédique selon la revendication 10, dans lequel l'angle latéral est situé entre 25 et 65 degrés.

12. Dispositif de support orthopédique selon la revendication 10 ou 11, dans lequel la main est supportée de manière à ce que le pouce soit élevé par rapport à l'extérieur de la main.

13. Dispositif de support orthopédique selon l'une quelconque des revendications précédentes, dans lequel des moyens de ventilation sont prévus.
